(19) **Europäisches Patentamt / European Patent Office / Office européen des brevets**

(11) **EP 4 255 289 B1**

(12) **EUROPEAN PATENT SPECIFICATION**

(45) Date of publication and mention
of the grant of the patent:
**09.07.2025 Bulletin 2025/28**

(21) Application number: **21810049.3**

(22) Date of filing: **23.11.2021**

(51) International Patent Classification (IPC):
**A61B 5/01** (2006.01)     **A61B 5/11** (2006.01)
**A61B 5/0537** (2021.01)     **A61B 5/00** (2006.01)

(52) Cooperative Patent Classification (CPC):
**A61B 5/01; A61B 5/0537; A61B 5/1101;**
**A61B 5/1118; A61B 5/1128; A61B 5/4266;**
**A61B 5/4842; A61B 5/7275; A61B 5/7278;**
A61B 5/1123; A61B 5/4839; A61B 5/7221;
A61B 5/7257; A61B 2560/0238; A61B 2560/0252

(86) International application number:
**PCT/EP2021/082571**

(87) International publication number:
**WO 2022/117392 (09.06.2022 Gazette 2022/23)**

(54) **MONITORING SHIVERING AND SWEATING EPISODES**

ÜBERWACHUNG VON ZITTER- UND SCHWITZEPISODEN

SURVEILLANCE D'ÉPISODES DE TREMBLEMENT ET DE SUDATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.12.2020 EP 20210971**

(43) Date of publication of application:
**11.10.2023 Bulletin 2023/41**

(73) Proprietor: **Koninklijke Philips N.V.
5656 AG Eindhoven (NL)**

(72) Inventors:
• **HUIJBREGTS, Laurentia Johanna**
**5656 AG Eindhoven (NL)**
• **JOHNSON, Mark Thomas**
**5656 AG Eindhoven (NL)**
• **VAN LIESHOUT, Ron Martinus Laurentius**
**5656 AG Eindhoven (NL)**
• **GERHARDT, Lutz Christian**
**5656 AG Eindhoven (NL)**
• **PELSSERS, Eduard Gerard Marie**
**5656 AG Eindhoven (NL)**

(74) Representative: **Philips Intellectual Property &
Standards
High Tech Campus 52
5656 AG Eindhoven (NL)**

(56) References cited:
EP-A1- 2 526 856     US-A1- 2018 242 850
US-A1- 2018 249 919     US-A1- 2019 117 155

**Description**

FIELD OF THE INVENTION

The present invention relates to a system and a computer program for monitoring shivering and sweating episodes in a patient.

BACKGROUND OF THE INVENTION

Fever is defined in a patient as the patient having a core body temperature (CBT) above the normal range due to an increase in the body's temperature set point. A fever can be caused by many medical conditions ranging from non-serious to life threatening. This includes viral, bacterial and parasitic infections such as the common cold, urinary tract infections, meningitis, malaria, and appendicitis among others. Non-infectious causes include vasculitis, deep vein thrombosis, side effects of medication, and cancer among others. Fever is considered advantageous to the host in surviving bacterial infections and is regulated by the thermoregulation methods of the body, where in particular sweating and shivering play a major role.

US 2018/0242850 discloses obtaining a perfusion parameter from temperature monitoring of a patient, and thereby determining a core body temperature, from which a user condition such as fever may be characterized.

WO 2017/172837 A1 discloses systems, methods and devices for utilizing heat transfer parameters or energy expenditure of devices providing controlled hypothermia, normothermia or hyperthermia to detect changes, or the absence of changes, to a patient's endogenous set-point temperature. When the body's core temperature deviates below an interthreshold zone, a series of coordinated responses occur that include surface vasoconstriction, shivering, and metabolic thermogenesis, among others. When the body's core temperature deviates above the interthreshold zone, a series of coordinated responses occur that include surface vasodilatation and sweating, among others.

Depending on the nature of the medical condition, the CBT of the patient may exhibit different time-dependent behavior. For example, fever linked to infection is caused by the increased set point due to cytokines released in the blood stream by circulating leukocytes, but when the source of infection, or in other words, when the activation of the host immune system is decreased, the concentration of cytokines in the blood stream decreases leading to a reduced inhibition in the hypothalamus, which leads to a lower set point and hence sweating to reach to lower set point, and as a consequence to a CBT that exhibits cyclic behavior of fever episodes over time. The CBT monitored over a prolonged period of time is also known as a fever curve, which can provide diagnostically relevant information to a medical professional such as a clinician or a nurse.

In high-acuity settings in the hospital, in particular in the Intensive Care Unit (ICU), CBT may be measured continuously. This is done via an invasive measurement. In lower-acuity settings, in particular on the general ward, it is desired to refrain from invasive techniques, e.g. because of infection risk. As there is no established reliable continuous non-invasive technique to measure CBT, CBT is measured during spot checks that commonly only take place one to three times per day. As a result, no fever curve can be reconstructed.

SUMMARY OF THE INVENTION

The present disclosure seeks to provide a non-invasive patient monitoring method.

The present invention further seeks to provide a computer program product for implementing such a method on a monitoring system, and a monitoring system for monitoring a patient, the monitoring system being arranged to implement such a method.

The invention is defined by the independent claims. The dependent claims define advantageous embodiments. The output may be a surrogate fever curve and/or other information that may be used by a healthcare professional to draw conclusions about the patient. The output based on the shivering and sweating episodes may be used for evaluation by a medical professional to obtain diagnostically relevant information about the progression of fever in the patient, which for instance may assist the medical professional in recognizing the type of medical condition of the patient, in defining a medication regime for the patient or in assessing the effectiveness of an applied medication regime, e.g. by monitoring changes in the surrogate fever curve after administration of the medication.

According to an embodiment, there is provided a computer-implemented method for monitoring progression of a fever in a patient, the computer-implemented method comprising, with a processor arrangement, receiving input data indicative of shivering episodes and sweating episodes of the patient as indicators of progression of a fever in the patient over a time interval; recording the respective points in time at which the shivering episodes and sweating episodes occur; and generating an output, e.g an output indicative of progression of fever in the patient, based on an alternating pattern of the shivering episodes and sweating episodes from the recorded respective points in time of the respective shivering and/or sweating episodes.

Embodiments of the present invention are based on the insight that detection of shivering and sweating episodes of a patient such as a patient or any other being at different points in time during a monitoring period, i.e. the time interval, can be interpreted as indicators of a change in CBT of the patient, i.e. the progression or change of a status of a fever in the patient, without having to record the actual CBT of the patient. **In** some embodiments, by recording the points in time at which these shivering and sweating episodes occur, a surrogate fever curve can be constructed for the patient. Such a fever curve may be considered a surrogate fever curve because the curve is not based on actual CBT measurements but rather is based on inferences of changes in the patient's CBT from the aforementioned detection of shivering and sweating episodes.

**In** an embodiment, the computer-implemented method further comprises, with the processor arrangement, determining a periodicity of the alternating pattern of the detected shivering and sweating episodes based on their respective occurrence at different points in time during the time interval. Based thereon, a healthcare professional may identify a type of fever from the determined periodicity of the alternating pattern. By identifying the type of fever, e.g. based on the time lapse between subsequent shivering episodes, subsequent sweating episodes and/or a shivering episode and a subsequent sweating episode or vice versa, additional diagnostically relevant information can be extracted from the surrogate fever curve and presented to the medical professional, e.g. to aid the medical professional in the effective treatment of the patient's medical condition.

Such points in time may be recorded by a user manually generating the input data, e.g. an acknowledgement signal or the like, during the occurrence of a shivering or sweating episode of the patient. However, in a particularly advantageous embodiment, receiving the input data comprises receiving patient monitoring data from a sensor arrangement for monitoring shivering and sweating of the patient as indicators of progression of a fever in the patient over the time interval; and processing the received patient monitoring data to detect shivering and sweating episodes and record the respective points in time at which the detected shivering episodes and patent sweating episodes occur in the patient monitoring data such that no manual inputs to monitor the patient are required.

The computer-implemented method may further comprise, with the processor arrangement, determining at least one of a duration and an intensity of each shivering episode and sweating episode; and optionally calculating an indication of an intensity of the fever from the determined at least one of a duration and an intensity of each shivering episode and sweating episode. This may provide valuable insights in the patient's ability to fight the fever, e.g. when caused by an infection, as expressed by the trends in the severity and/or the duration of such episodes over time.

In a further refinement, the patient monitoring data may comprise information pertaining to a degree of thermal insulation of the patient, and the computer-implemented method may further comprise, with the processor arrangement, determining at least one of a duration and a severity of each shivering episode and sweating episode as a function of the information pertaining to a degree of thermal insulation of the patient. For example, whether the patient is covered by one or more blankets and/or wears a certain amount of clothing may have an impact on the severity and duration such that detection of these external influences can be used to more accurately assess the true nature of the shivering or sweating episode.

In a particular embodiment, the computer-implemented method further comprises, with the processor arrangement, determining a shivering frequency for each shivering episode; and determining if the shivering episode is an indicator of an illness-induced body temperature change in the patient based on the determined shivering frequency. This is based on the insight that the shivering frequency caused by the onset of fever differs from the frequency of other involuntary shivering-like body motions, e.g. tremors or the like, thereby further ensuring that the shivering episodes included in the surrogate fever curve are genuinely associated a change in a patient's CBT caused by the onset of fever.

Preferably, a sweating episode is identified based on a sweat rate of the patient as this is a particularly accurate way of determining such a sweating episode. For example, the computer-implemented method may further comprise, with the processor arrangement, determining a sweat base rate for the patient from the received patient monitoring data during a shivering episode; and detecting a sweating episode by detecting an actual sweat rate of the patient in the received patient monitoring data that exceeds the sweat base rate by a defined amount. This is particularly advantageous as it provides a patient-specific baseline for the sweat rate based on the insight that during a shivering episode the patient's sweat rate is typically minimal, such that any sweat rate that is significant to the patient can be detected by comparing an actual sweat rate against this base rate, thereby making the detection method robust against varying sweat rates between patients.

Alternatively or additionally, the computer-implemented method may comprise, with the processor arrangement, comparing an actual sweat rate of the patient against a defined sweat rate threshold; and detecting a sweating episode upon the actual sweat rate exceeding the defined sweat rate threshold to ensure that the detected sweat rate is sufficiently high such that its detection can be associated with a genuine sweating episode indicative of a lowering of the patient's CBT, i.e. the subsidence of a fever in the patient.

In a further embodiment, the computer-implemented method further comprises, with the processor arrangement, determining a signal strength of a signal pertaining to shivering episodes over the time interval; determining a total amount of sweat produced by the patient during the sweating episodes over the time interval; determining the patient's sweat rate over the time interval from the determined total amount of sweat produced by the patient; calculating a ratio of the determined signal strength and determined sweat rate; and including the calculated ratio in the output. This ratio may be used to reconstruct the surrogate fever curve to enable prediction of the severity of the fever and medication response efficacy for example.

The patient monitoring data may further comprise core body temperature data pertaining to the patient's core body temperature measured over the time interval with a core body temperature sensor of the sensor arrangement, wherein the computer-implemented method may further comprise, with the processor arrangement, including the patient's core body temperature data in the output. This for example allows for the determination of a lag between the occurrence of a shivering or sweating episode and the associated change in the patient's CBT as monitored by the core body temperature sensor, from which the patient's thermoregulatory ability can be derived. Moreover, the CBT measurement may assist in distinguishing between a shivering episode caused by the onset of fever, where this causes the patient's CBT to increase and a shivering episode caused by the patient being cold, where the shivering episode will not lead to an increase in the patient's temperature set point. This for instance may be used to verify whether the shivering episode can be reliably associated with the start of a fever episode, thereby further improving the accuracy of the patient monitoring. Moreover, inclusion of the CBT information, e.g. over a limited amount of time such as a time period covering a few fever cycles can be used for calibrating or training the construction of the surrogate fever curve by the processor arrangement. For example, the CBT data may be used as a ground truth to train artificial intelligence or machine learning algorithms. Such calibration or training thus can lead to the generation of a surrogate fever curve in which actual CBT can be accurately estimated. Such CBT information for example may be collected during spot checks or when the patient is being continuously monitored in an intensive care setting such as during an invasive procedure.

**In** another embodiment, the computer-implemented method further comprises, with the processor arrangement, receiving auxiliary information during the time interval through the communication interface arrangement, the auxiliary information comprising at least one of an ambient temperature to which the patient is exposed and a measurement of an activity level of the patient; determining whether a shivering episode or a sweating episode is a reliable indicator of an illness-induced body temperature change in the patient based on the actual auxiliary information at the point in time of the shivering episode or sweating episode; and including the shivering episode or a sweating episode in generating the output only when the shivering episode or a sweating episode is determined to be a reliable indicator of progression of a fever in the patient. This facilitates the exclusion of shivering or sweating episodes that are triggered by factors other than fever from the surrogate fever curve, thereby increasing the accuracy of the surrogate fever curve.

According to another aspect, there is provided a computer program product comprising a computer readable storage medium having computer readable program instructions embodied therewith for, when executed on a processor arrangement of a monitoring system of a patient, cause the processor arrangement to implement the method of any of the herein described embodiments. Such a computer program product may be used to configure a monitoring system such that it can implement the method according to embodiments of the present invention.

According to yet another aspect, there is provided a monitoring system for monitoring a patient, the monitoring system comprising a processor arrangement arranged to receive input data indicative of shivering episodes and sweating episodes of the patient as indicators of progression of a fever in the patient over a time interval, wherein the monitoring system further comprises the computer program product of any of the herein described embodiments and the processor arrangement is arranged to execute the computer readable program instructions. Such a monitoring system therefore is capable of generating a surrogate fever curve of a patient without having to collect CBT information from the shivering and sweating episodes of the patient as captured in the patient monitoring data provided by the sensor arrangement, in which the monitoring system may provide the output based on the surrogate fever curve, e.g. an output including the surrogate fever curve and/or diagnostically relevant information derived from the shivering and sweating episodes, to a user interface such that the output may be assessed on the user interface, e.g. by a medical professional, or may store the output in a data storage architecture such as an electronic patient record for retrieval at any suitable point in time, e.g. by a medical professional.

The monitoring system may further comprise a communication interface arrangement arranged to receive the input data in the form of patient monitoring data from a sensor arrangement for monitoring shivering and sweating as indicators of progression of a fever in the patient and to forward the patient monitoring data to the processor arrangement, the monitoring system optionally comprising the sensor arrangement such that the patient may be monitored without requiring manual intervention, thereby providing a complete solution for patient fever progression monitoring based on the monitoring of the patient's shivering and sweating episodes as explained above. The sensor arrangement may comprise at least one of a camera, a sensor pad for integration in a bed, a wearable sensor including a sweat sensor and a motion sensor for detecting shivering, and preferably the sensor arrangement comprises the wearable sensor.

BRIEF DESCRIPTION OF THE DRAWINGS

Embodiments of the invention are described in more detail and by way of non-limiting examples with reference to the accompanying drawings, wherein:

FIG. 1 schematically depicts a monitoring system according to an embodiment;
FIG. 2 schematically depicts an example fever curve (core body temperature on y-axis, time on x-axis);
FIG. 3 schematically depicts an aspect of the monitoring system of FIG. 1 in more detail;
FIG. 4 shows a flowchart of a fever progression monitoring method according to an embodiment;
FIG. 5 depicts a graph explaining the construction of a surrogate fever curve according to an example embodiment;
FIG. 6 depicts a graph explaining the construction of a surrogate fever curve according to another example embodiment;
FIG. 7 depicts a graph explaining the construction of a surrogate fever curve according to yet another example embodiment;
FIG. 8 shows a flowchart of an aspect of a fever progression monitoring method according to an embodiment; and
FIG. 9 is a graph (reconstructed core body temperature index on y-axis, time on x-axis) depicting several fever index curves.

DETAILED DESCRIPTION OF THE EMBODIMENTS

It should be understood that the Figures are merely schematic and are not drawn to scale. It should also be understood that the same reference numerals are used throughout the Figures to indicate the same or similar parts.

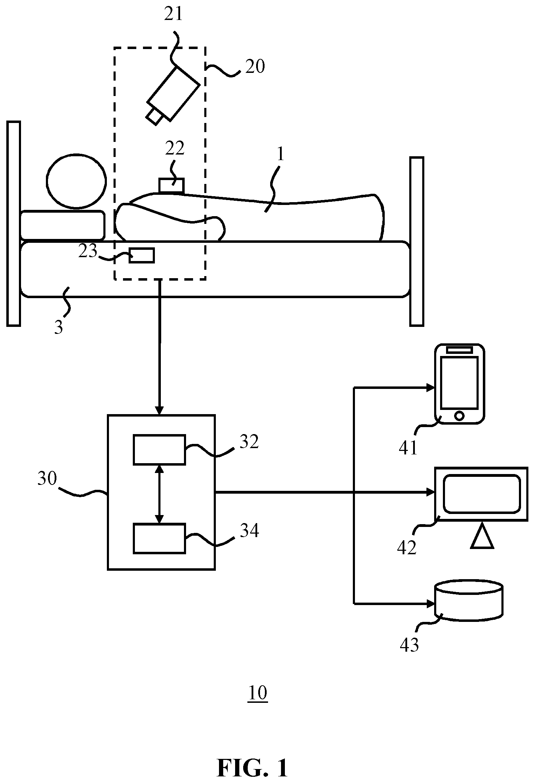

FIG. 1 schematically depicts an arrangement for monitoring the progression of a fever in a patient 1 with a monitoring system 10. The monitoring system 10 typically comprises a data processing architecture 30, e.g. a computer system, server system or the like, including a communication interface arrangement 32 in communication with a processor arrangement 34. The communication interface arrangement 32 may comprise any suitable number of data communication interfaces such as one or more P2P communication interfaces such as a Bluetooth interface, one or more wireless communication interfaces such as a WiFi interface, one or more wired communication interfaces such as an Ethernet interface, e.g. for connecting the data processing architecture 30 to a local area network, the Internet or the like, and so on. The processor arrangement 34 may comprise one or more processing elements such as one or more processors, processor cores and the like, arranged to process patient monitoring data provided by the sensor arrangement 20.

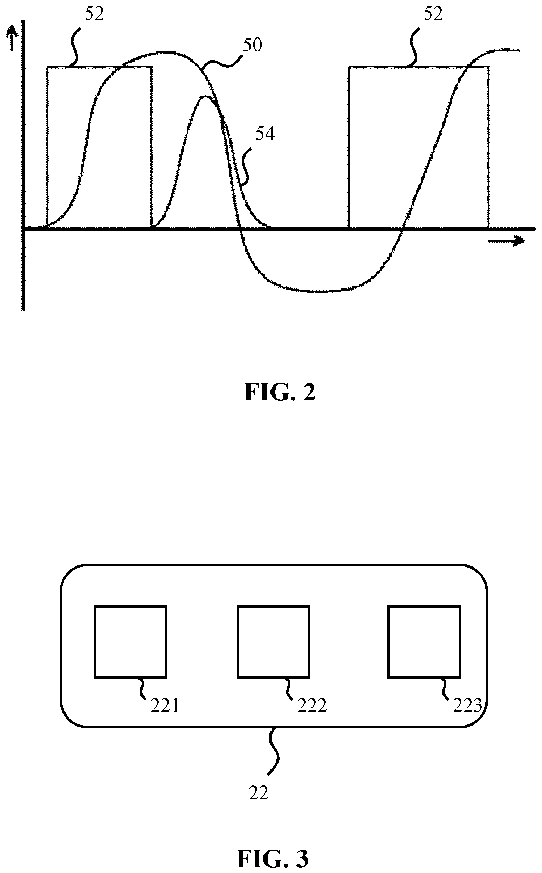

The sensor arrangement 20, which may form part of the monitoring system 10, is typically arranged to provide patient monitoring data that allows the processor arrangement 34 to detect shivering episodes 52 and sweating episodes 54 of the patient 1 being monitored in order to allow the processor arrangement 34 to construct a surrogate fever curve 50 for the patient 1 as schematically depicted in FIG. 2. This will be explained in further detail below. The patient monitoring data is typically provided to the data processing architecture 30 over more or more data communication links between the sensor arrangement 20 and the communication interface arrangement 32, which communication interface 32 forward the received patient monitoring data to the processor arrangement 34. In order to provide patient monitoring data from which the processor arrangement 34 can derive shivering and sweating episodes of the patient 1, the sensor arrangement 20 may comprise one or more sensors for this purpose.

For example, the sensor arrangement 20 may include a camera 21 aimed at the patient 1 to detect patient shivering, e.g. through the detection of patient motion in the images provided by the camera 21, and sweating, e.g. through changes in light reflection caused by the formation of sweat droplets on an exposed part of the patient's skin such as the patient's forehead as detected in the images provided by the camera 21. The sensor arrangement 20 may comprise a wearable sensor 22 that may be attached to the patient's skin for providing the required patient monitoring data. An example embodiment of such a wearable sensor 22 is shown in FIG. 3, which depicts a wearable sensor 22 comprising a motion sensor 221 such as an EMG or ECG sensor, an accelerometer or the like through which patient motion, e.g. shivering, can be detected, a sweat sensor 222 through which sweat production in a region of the patient's skin to which the wearable sensor 22 is attached can be detected, and optionally at least one additional sensor 223, for providing additional sensor data as will be explained in further detail below.

The sweat sensor 222 may be arranged to measure a sweat flow rate or a sweat volume after a defined collection time, or alternatively may be arranged to measure a sweat parameter indirectly, e.g. by measuring skin conduction. The wearable sensor 22 when including both the motion sensor 221 and the sweat sensor 222 should be placed on a part of the patient's body where both shivering and sweating can be reliably measured, such as on the patient's thorax or neck. The sensor arrangement 20 may comprise a sensor 23 for integration in the bedding, e.g. the mattress 3 on which the patient 1 is lying, for detection of patient motion whilst the patient 1 is in the bed, from which shivering may be detected. Any suitable combination of such sensors 21, 22, 23 may be deployed. In a preferred embodiment, the sensor arrangement 20 at least comprises the wearable sensor 22.

The data processing architecture 30 may further be communicatively coupled to one or more user interfaces 41, 42, on which the processing result of the patient monitoring data processing by the processor arrangement 34, e.g. the surrogate fever curve 50, may be displayed. To this end, the processor arrangement 34 typically generates an output based on this processing result, e.g. including this processing result or information derived from this processing result, which output may be forwarded to a particular user interface 41, 42 via the communication interface arrangement 32. For example, the user interface 41 may be a mobile communication device comprising an app or the like for visualizing the processing result of the processor arrangement 34, which may be used by a medical professional such as a nurse or doctor to evaluate the fever of the patient 1. The user interface 42 may be a user terminal such as a personal computer or the like through which the medical professional may receive the processing result from the processor arrangement 34. Alternatively or additionally, the processor arrangement may be arranged to store the processing result in a data storage arrangement 43, e.g. a network-connected database or the like storing the patient's monitoring data, e.g. an electronic patient record or the like, such that the processing result may be accessed at any suitable point in time, e.g. using the user interface 41 or 42. Other suitable architectures will be apparent to the skilled person.

In an alternative embodiment (not shown), the sensor arrangement 20 may be omitted and the processor arrangement 34 may instead be arranged to receive manually generated input data, e.g. by the patient 1 or by a medical caregiver such as a nurse or doctor, using an input device such as a remote control, an electric communication device such as a smart phone, or the like, such that the person operating such an input device can signal the occurrence of a shivering episode 52 or a sweating episode 54 of the patient 1 with the input device, such that the processor arrangement only has to record the point in time at which such an input has been received to construct the surrogate fever curve 50.

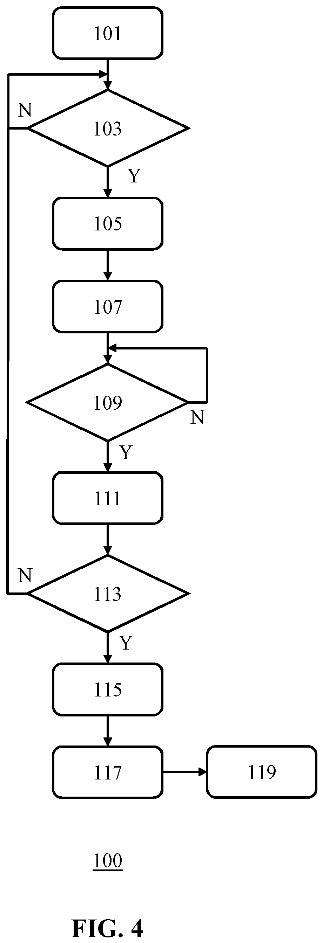

The monitoring system 10 is arranged to implement one or more of the embodiments of the method 100 of the present invention, a flowchart of which is shown in FIG. 4. The method 100 starts in operation 101, for example in which the sensor arrangement 20, if present, is activated and provides the previously described patient monitoring data to the processor arrangement 34 through the communication interface arrangement 32. In operation 103, the processor arrangement 34 processes the patient monitoring data received from the sensor arrangement 20 to determine if a shivering episode 52 of the patient 1 can be detected in the patient monitoring data. This processing may take place continuously or periodically, i.e. the patient monitoring data may be processed on a continuous basis or may be sampled periodically, e.g. once every 1- 5 minutes, for processing by the processor arrangement 34. If no shivering is detected, the processor arrangement 34 continues to look for a shivering episode 52 of the patient 1 in the received patient monitoring data. However, if the processor arrangement 34 detects the occurrence of such a shivering episode 52, the method proceeds to operation 105 in which the processor arrangement records the point in time at which the shivering episode 52 was detected, e.g. as derived from the patient monitoring data provided by the sensor arrangement 20 or as derived from manually generated input data flagging a shivering episode 52. This point in time may be expressed in any suitable format, e.g. as the amount of time lapsed after commencement of the patient monitoring, as the actual time of day or the like.

The method 100 may proceed to operation 107, in which the processor arrangement 34 determines a sweat base rate for the patient 1 during the shivering episode 52. This is based on the insight that during such a shivering episode 52, the CBT, i.e. the temperature set point, of the patient 1 is increasing, which typically equates to minimal associated sweating. Therefore, determining a sweat rate during such a shivering episode 52 provides a reliable baseline for determining a subsequent sweating episode 54 of the patient 1, as an actual sweating episode 54 of the patient 1 would lead to a significantly higher sweat rate than the baseline sweat rate. The determination of such a baseline sweat rate has the advantage that a patient-specific sweat rate baseline is defined, such that there is no need to account for different sweat rates in different patients in order to reliably detect a sweating episode of the patient 1. However, in alternative embodiments the detection of such a sweat base rate may be omitted, in which case a sweating episode 54 in a patient 1 may be detected using a defined sweat rate threshold, which may be generic, gender-specific, specific to a location of the wearable sensor 22 on the patient's body, and so on.

In operation 109, the processor arrangement 34 processes the patient monitoring data received from the sensor arrangement 20 to determine if a sweating episode 54 of the patient 1 can be detected in the patient monitoring data. This processing may take place continuously or periodically, i.e. the patient monitoring data may be processed on a continuous basis or may be sampled periodically, e.g. once every 1 to 5 minutes, for processing by the processor arrangement 34. For example, the processor arrangement 34 may determine an actual sweat rate of the patient 1 and may compare this actual sweat rate against the sweat base rate determined in operation 107 and determine that a sweating episode 54 is occurring if the actual sweat rate exceeds the sweat base rate by a defined amount, e.g. a defined factor such as a factor 4-6.

Alternatively or additionally, the processor arrangement 34 may compare the actual sweat rate against a defined sweat rate threshold and determine that a sweating episode 54 is occurring if the actual sweat rate exceeds the defined threshold to ensure that the sweat rate is sufficiently high, e.g. above 0.7 nL/min for a sweat gland, to decide that an sweating episode 54 is occurring with sufficiently high confidence. Such a sweat rate may be determined using the wearable sensor 22, e.g. by determining an actual sweat rate or a parameter associated with changes in sweat rate such as skin conductance, or with the camera 21, e.g. by determining a change in reflected light from the camera images and deriving the sweat rate from this change.

If no sweating episode 54 is detected, the processor arrangement 34 continues to look for a sweating episode 54 of the patient 1 in the received patient monitoring data in operation 109. However, if the processor arrangement 34 detects the occurrence of such a sweating episode, e.g. in the patient monitoring data provided by the sensor arrangement 20 or as derived from manually generated input data flagging a sweating episode 54, the method proceeds to operation 111 in which the processor arrangement 34 records the point in time at which the sweating episode 54 was detected. This point in time may be expressed in any suitable format, e.g. as the amount of time lapsed after commencement of the monitoring of the patient 1, as the amount of time lapsed after the previously detected shivering episode 52, as the actual time of day, or the like.

As expressed in operation 113, the recording of the points in time at which shivering episodes 52 and sweating episodes 54 may continue, in which case the method 100 returns to operation 103 in which the processor arrangement 34 processes the patient monitoring data to detect the next shivering episode 52 or alternatively awaits the manual flagging of such an episode as previously explained. The method 100 may instead proceed to operation 115 in which the processor arrangement 34 constructs the surrogate fever curve 50 for the patient 1 from the recorded points in time at which the respective shivering episodes 52 and sweating episodes 54 of the patient 1 occurred. It is noted for the avoidance of doubt that the construction of the surrogate fever curve 50 may be performed after monitoring the patient 1 over a certain time interval, or alternatively the construction of the surrogate fever curve 50 may be performed in parallel with the monitoring the patient 1 over the time interval. The processor arrangement 34 may construct the surrogate fever curve 50 based on the alternating pattern of shivering episodes 52 and sweating episodes 54 and the periodicity of this alternating pattern.

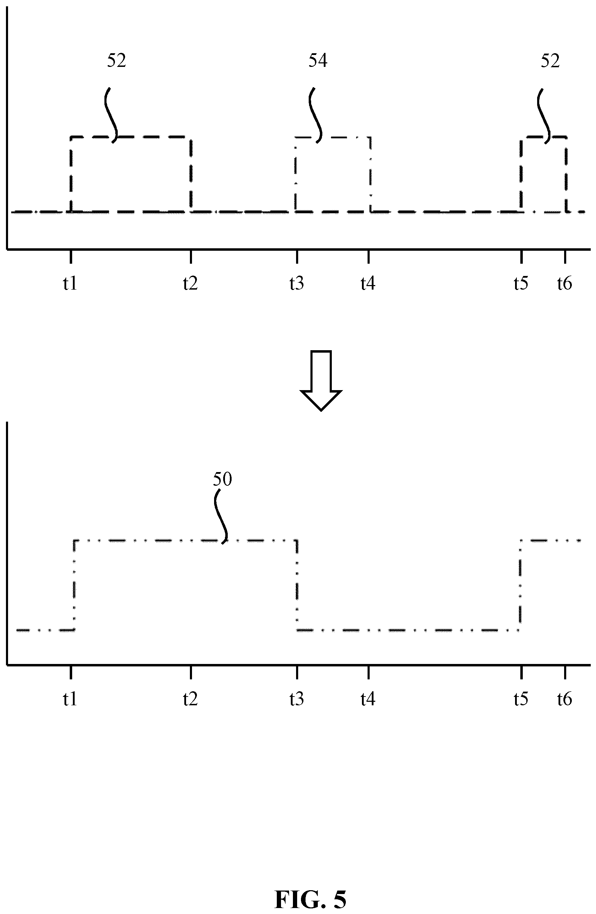

For example, as schematically depicted in FIG. 5, the processor arrangement 34 may receive an indication of the onset of a shivering episode 52 at T = t1, e.g. from the sensor arrangement 20 or through a manual input as previously explained, which shivering episode 52 terminates at T = t2. The upper graph shows intensity (a.u.) of the shivering episodes 52 and sweating episodes 54 (Y-axis) as a function of time (X-axis). The processor arrangement 34 further receives an indication of the onset of a sweating episode 54 at T = t3, e.g. from the sensor arrangement 20 or through a manual input as previously explained, which sweating episode 54 terminates at T = t4. The processor arrangement 34 further receives an indication of the onset of a further shivering episode 52 at T = t5, e.g. from the sensor arrangement 20 or through a manual input as previously explained, which further shivering episode 52 terminates at T = t4. The processor arrangement 34 may construct the surrogate fever curve 50 from the points in time at which a shivering episode 52 and a sweating episode 54 initiated, i.e. t1, t3 and t5 without factoring in the duration of each episode, which produces the resulting surrogate fever curve 50 as shown in FIG. 5, with CBT, or more accurately, projected change in CBT (Y-axis) as a function of time (X-axis). As can be seen from this curve, the onset of each shivering episode 52 is interpreted as an instantaneous rise in CBT, whereas the onset of each sweating episode in interpreted as an instantaneous drop in CBT. Of course, alternative approaches, e.g. the fitting of a sinusoidal curve based on the recorded points in time of the onset of respective shivering episodes 52 and sweating episodes 54 are also feasible. The processor arrangement 34 subsequently generates an output based on the thus constructed surrogate fever curve 50 in operation 117, e.g. for transmission to the user interface 41 or 42 and/or for storage in the data storage architecture 43, after which the method 100 terminates in operation 119.

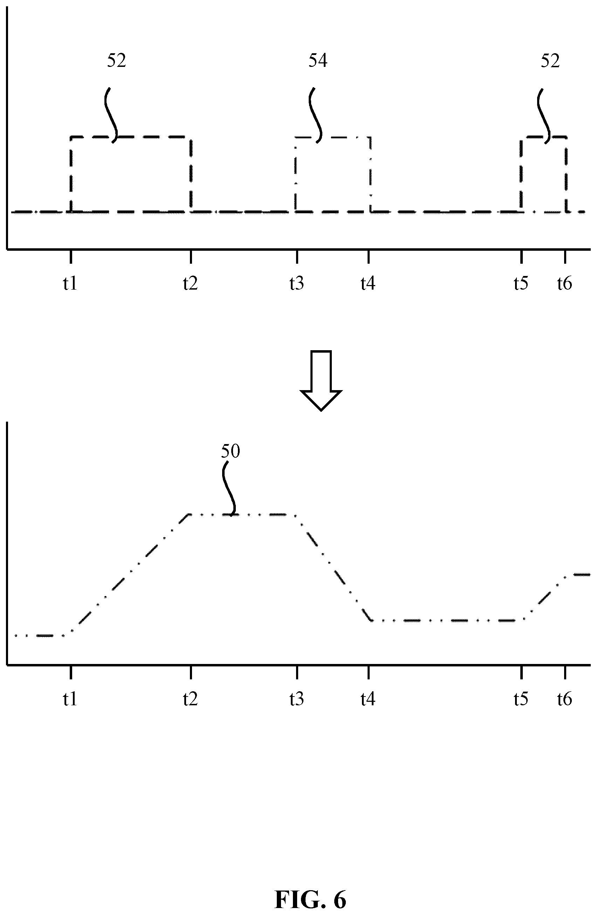

In a refinement, when constructing the surrogate fever curve 50 in operation 115, the processor arrangement 34 may factor in the duration of each of the shivering episodes 52 and sweating episodes 54. This is shown in FIG. 6, in which at the start of each shivering episode 52, the processor arrangement 34 assumes a gradual increase such as a linear increase of the CBT of the patient until the end of such a shivering episode 52, after which the CBT is assumed constant until the start of a subsequent sweating episode 54, at which point in time the processor arrangement 34 assumes a gradual decrease such as a linear increase of the CBT of the patient until the end of such a sweating episode 54 in its construction of the surrogate fever curve 50. Again, as an alternative, a sinusoidal curve may be fitted based on the start and end values of the respective start and end points (in time) of the respective shivering episodes 52 and sweating episodes 54.

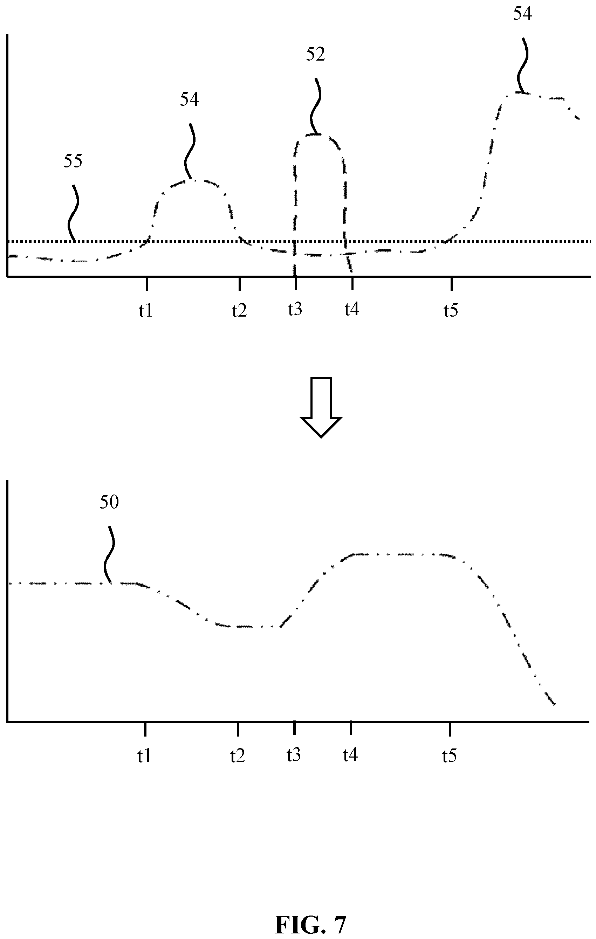

In a further refinement, as shown in FIG. 7, the processor arrangement 34 may take both the duration and the intensity of the shivering episodes 52 and sweating episodes 54 into consideration in the construction of the surrogate fever curve 50. In this approach, when the patient 1 is sweating heavily, i.e. has a high sweat rate, the reproduced approximation of the CBT of the patient 1 decreases faster than when the patient 1 sweats only a little, i.e. has a low sweat rate. As mentioned above, a sweating episode 52 may be detected upon the actual sweat rate exceeding a sweat base rate or threshold 55. Similarly, when the patient 1 is shivering heavily, the reproduced approximation of the CBT of the patient 1 increases faster than when the patient 1 only shivers a little. The intensity of a shivering episode may be determined using a power spectral density analysis as will be explained in more detail below. Since the intensity level of a shivering episode 52 or a sweating episode 54 can change over time, instead of the binary an/off approach in the construction of the surrogate fever curve 50 in FIG. 5, it thus becomes an integral as expressed in equation (1):

$$'temperature'(t) = T_0 + \int_0^t (c_1(intensity\ shivering) - c_2(intensity\ sweating))dt \qquad (\text{equation 1})$$

In this equation, t is the time, T0 is the temperature at t=0, which may be measured, estimated, or may be a default value, and c1 and c2 are parameters that are patient- and environment- dependent (e.g. factoring in the degree thermal insulation of the patient 1, *vide infra*) and may even depend on the intensity of respectively shivering and sweating. If no independent CBT measurement is available, default values may be used for T0, c1 and c2. However, if such an independent CBT measurement becomes available, this measurement may be used to determine T0. Where multiple independent CBT measurements over a period of time become available, e.g. through continuous or spot check CBT measurements over a period of time during which shivering episodes 52 and sweating episodes 54 occur, c1 and c2 can also be estimated for the particular patient, environment, and shivering/sweating episode intensity levels during the CBT measurements. This may serve as a calibration of the surrogate fever curve 50 construction by the processor arrangement 34 once the CBT measurements have stopped. Hence, in this embodiment it is possible to provide a surrogate fever curve 50 including estimated absolute values for the patient's CBT on the Y-axis of the graph depicting the surrogate fever curve 50 as a function of time on the X-axis of this graph.

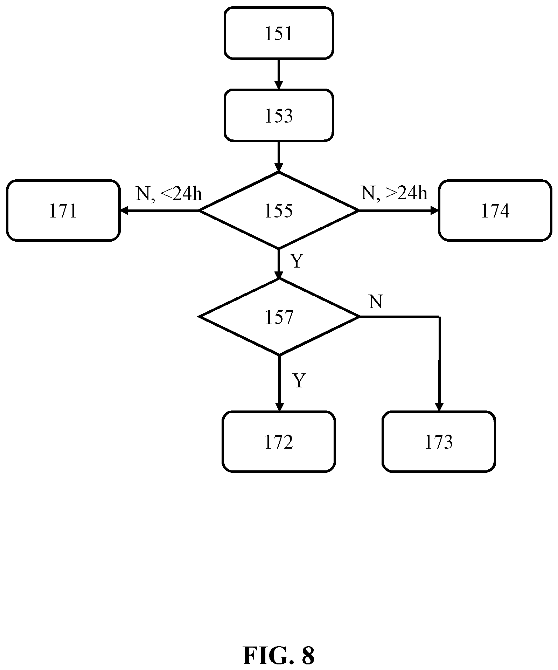

In a preferred embodiment, the processor arrangement 34 generates information assisting a healthcare professional to treat the patient, including determining the type of fever from the recorded points in time at which the detected shivering episodes 52 and the sweating episodes 54 of the patient 1 occur, e.g. in addition to or instead of the generation of the surrogate fever curve 50, and includes this information in the output generated in operation 117, e.g. in addition to the surrogate fever curve 50 or as information derived from the surrogate fever curve 50 without including the actual surrogate fever curve 50, to provide a medical professional with additional insights in the underlying condition of the patient 1. This is explained in more detail with the aid of FIG. 8, which schematically depicts a decision tree that may be deployed by the processor arrangement 34 for determining the type of fever from the recorded points in time. The decision tree starts in operation 151, after which the processor arrangement 34 in operation 153 determines the periodicity of the surrogate fever curve 50 in operation 153, e.g. by determining the elapsed time between successive shivering episodes 52 separated by a sweating episode 54 or between successive sweating episodes 54 separated by a shivering episode 52 from the recorded points in time. If this periodicity is less than about 24 hours, as determined in operation 155, the processor arrangement 34 determines that the type of fever is a remittent fever, as symbolized by conclusion 171.

If on the other hand it is determined that this periodicity is about 24 hours, the processor arrangement 34 decides that the type of fever is a quotidian fever. The processor arrangement 34 may then further evaluate the elapsed time between a shivering episode 52 and a subsequent sweating episode 54 in operation 157 to determine the ability of the patient's body to fight the quotidian fever. Typically, when the patient's immune system is functioning properly, the duration of the elevated CBT or fever should be no longer than a few hours, during which the burst of parasites is attached by the patient's immune system. Hence, when the processor arrangement 34 concludes in operation 157 that the duration of a period of elevated CBT corresponds to an appropriate functioning immune system, the processor arrangement 34 may conclude that the fever is 'normal' quotidian fever as symbolized by conclusion 172, whereas if the processor arrangement 34 concludes in operation 157 that the duration of a period of elevated CBT exceeds a threshold value indicative of a compromised immune system, the processor arrangement 34 may conclude that the fever is quotidian fever in a weakened patient 1, as symbolized by conclusion 173.

Of course, the evaluation of the state of the patient's immune system is optional, in which case the determination of the elapsed time between a shivering episode 52 and a subsequent sweating episode 54 may be omitted from this evaluation. Consequently, recording of the points in time at which the sweating episodes 54 occur may be omitted and the evaluation of the surrogate fever curve 50 may be based on the elapsed time between successive shivering episodes 52 only, as long as these shivering episodes are separated by a sweating episode 54. Equally, recording of the points in time at which the shivering episodes 52 occur may be omitted and the evaluation of the surrogate fever curve 50 may be based on the elapsed time between successive sweating episodes 54 only, as long as these sweating episodes are separated by a shivering episode 52.

If the processor arrangement 34 determines in operation 155 that the periodicity of the fever in the patient 1 clearly exceeds 24 hours, the processor arrangement 34 may conclude that the fever is another type of fever as symbolized by conclusion 174, such as a tertian fever, a quartan fever, relapsing fever, undulant fever or the like. A further decision tree (not shown) may be implemented by the processor arrangement 34 to distinguish between such further types of fever.

In at least some embodiments in which the sensor arrangement 20 is used to collect patient monitoring data from which the surrogate fever curve 50 is constructed as explained above, additional sensors, e.g. additional sensors 223, may be used for a number of reasons. For example, the sweat sensor 222 of the wearable sensor 22 may further be configured to determine the concentration of an analyte of interest, e.g. cytokines or lactate, in the excreted sweat, which may be used by the processor arrangement 34 to further determine the cause of the fever, the severity of the disease, and/or efficacy of administered medication to combat the disease, which information can provide helpful insights for the medical caregiver in how to treat the patient 1, e.g. by administering different medication dosages, different medication and so on. Further additional sensors, which preferably but not necessarily are integrated in one or more wearable sensors 22, may provide physiological monitoring data such as heart rate, respiration rate, blood pressure, peripheral oxygen saturation and so on, which sensor data may also be included in the output generated by the processor arrangement 34 to further enhance the diagnostic potential of this output.

The sensor arrangement 20 may further comprise one or more additional sensors that provide auxiliary information to the processor arrangement 34 that allows the processor arrangement 34 to distinguish between fever-induced shivering episodes 52 and sweating episodes 54 or the occurrence of such episodes for different reasons, e.g. one or more motion sensors, heart rate monitors, blood pressure sensors or the like to detect increased patient activity, e.g. sports activity, strenuous activity such as walking up stairs and the like, which can cause sweating episodes that are triggered by the activity rather than a fever-induced lowering of the patient's temperature set point. An environmental sensor, e.g. an ambient temperature sensor or the like may be deployed to determine if the onset of a shivering or sweating episode is caused by environmental factors, e.g. a low or high ambient temperature, instead of because of illness. Using this auxiliary information, the processor arrangement 34 may determine whether a shivering episode 52 or a sweating episode 54 is a reliable indicator of an illness-induced body temperature change of the patient 1 such that the processor arrangement 34 only includes the point in time of such a shivering episode 52 or sweating episode 54 in the surrogate fever curve 50 if the processor arrangement 34 decides that the shivering episode 52 or the sweating episode 54 is a reliable indicator of an illness-induced body temperature change of the patient 1 rather than likely to be caused by other factors as signaled by the auxiliary information.

In yet another embodiment, the sensor arrangement 20 further comprises a CBT sensor, which may be an additional sensor 223 in the wearable sensor 22 or a separate (wearable) sensor. Including CBT information in the patient monitoring data allows for even more information to be obtained from the points in time at which shivering episodes 52 and/or sweating episodes 54 occur. Typically, CBT will go up due to shivering and down due to sweating, but there is a certain lag time between the occurrence of such a shivering or sweating episode and the accompanying change in CBT. This lag time can be used to get an insight in the patient's thermoregulatory ability. In addition, the CBT information may be used to calibrate the construction of the surrogate fever curve 50 by the processor arrangement 34, e.g. as a ground truth for training artificial intelligence or machine learning algorithms used by the processor arrangement 34 to construct the surrogate fever curve 50.

Another reason to add a CBT measurement is to distinguish between shivering due to an elevated set point (resulting in fever) and shivering due to the patient 1 being too cold (with a normal set point). This is in particular useful when establishing the start of a fever period. One easy way to distinguish between these two possibilities is to perform a single CBT measurement when the shivering has been detected for a suitable period of time (say 10 minutes). If the elevated set point is the cause, the temperature will already be elevated, whilst if the body is cold it will be depressed.

As is well-known per se, fever is the increase of body temperature during an infection. However, the mechanism underlying this fever is more complex and involves the host response to the infection. The immune system is activated by the source of infection and releases chemokines in the body resulting into an activation of cyclooxygenase (COX) resulting in a stimulation of 'heat gain thermo-effectors' (i.e. shivering) and an inhibition of 'heat loss thermo-effectors' (i.e. sweating). Normally only the core-body temperature is measured, and apart from the aforementioned impractical clinical integration, it only provides information of the increase of the patient's temperature set point. Therefore, in a preferred embodiment, the construction of the surrogate fever curve 50 further includes the determination of the shivering rate and the sweat rate of the patient 1 to provide an additional layer of diagnostically relevant information to the surrogate fever curve 50 as this for example provides information about the host's response, i.e. the immune response, to the source of the infection. In addition or as an alternative to the measurement of the CBT of the patient 1 to determine the lag between the shivering and sweating episodes and the induced changes in the patient's CBT as explained above, such a host response may be calculated from a number of parameters, such as the changes in the amount of time between successive shivering episodes 52, the changes in the amount of time between successive sweating episodes 54, the changes in the amount of time between a shivering episode 52 and a subsequent sweating episode 54 as well as the intensity and duration of such shivering episodes 52 and sweating episodes 54. The intensity of a sweating episode 54 may be based on a sweat rate determined with the sensor arrangement 20. The intensity of a shivering episode 52 may be determined based on a Fourier analysis and/or a spectral power analysis of the shivering data provided by the sensor arrangement 20. This may serve a number of purposes. Firstly, such an analysis of the shivering data provides a measurement of the trembling frequency of the patient 1, which allows the processor arrangement to make a distinction between unintended muscle movements, e.g. age-related tremors or the like, and shivering due to an elevated set point compared to the actual CBT of the patient 1. For example, hand tremors caused by Parkinson's disease can be as high as 9 Hz, which tremor amplitudes and acceleration amplitudes can reach 19 cm and 20 m/s$^2$ respectively. During fever or hyperthyreoses, the frequency increases up to 14 Hz, thereby allowing a differentiation between fever-related tremors and other types of tremors, such that the processor arrangement 34 can discard shivering episodes 52 caused by such other type of tremors and only consider fever-induced shivering episodes 52 in the construction of the surrogate fever curve 50, e.g. by using a bandpass filter or a high-pass filter with a suitable cut-off frequency, e.g. 10 Hz.

Secondly, the ratio of the signal strength of the shivering signal produced by the sensor arrangement 20 to the total sweat production or the sweat rate over time as monitored over a patient monitoring time interval may be used to construct a surrogate fever curve to enable prediction of fever severity and medical therapy response efficacy.

In a first step, fast Fourier analysis, e.g. amplitude or power spectrum signal analysis, of the shivering signal is performed to determine the strength of shivering. The power spectral density (PSD) of the shivering signal (PSD_Shivering) describes the power present in the signal as a function of frequency, per unit frequency. Power spectral density is commonly expressed in watts per Hertz (W/Hz). The total power present in the signal over a relevant frequency range (e.g. 10-20 Hz, or 5-15 Hertz) is then calculated.

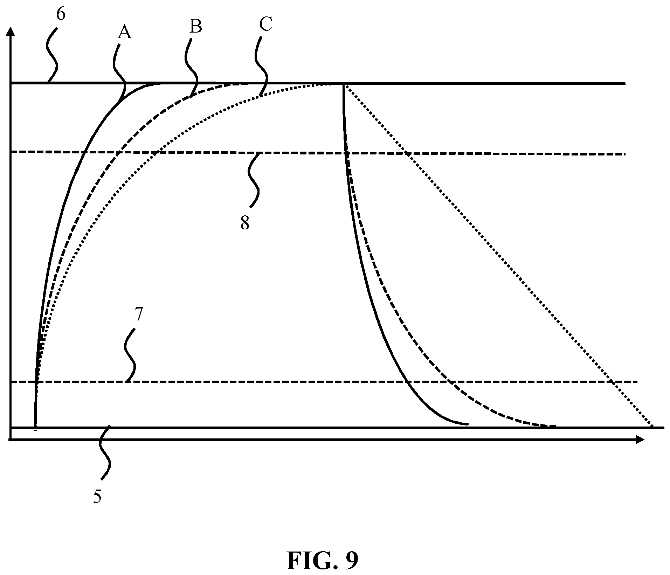

In a second step, the ratio of the shivering signal strength to the sweat rate (nL/s] is determined to calculate a fever index as per equations 2 and 3 below. Such a fever index may be an index at a given time (equation 2) or may be a time-integrated index (equation 3), i.e. the area under a fever index curve as shown in Fig. 9. If the fever index is integrated over time to calculate an accumulated fever index, (equation 2), this time-integrated index will provide clinical information pertaining to the fever episode or the fever interval intensity for a given time window of clinical interest. Another option is to calculate the first derivative of the fever index curve, which will then give information on the fever dynamics such as the speed of fever development or fever recovery.

$$Fever\ index\ \left[\frac{\frac{W}{Hz}}{\frac{nL}{s}}\right] = \frac{PSD\_Shivering}{Sweat\ rate} \qquad\qquad \text{(equation 2)}$$

$$Fever\ area\ index\ \left[\frac{\frac{W}{Hz}}{\frac{nL}{s}} \cdot s\right] = \int_{t=o}^{T} \frac{PSD\_Shivering}{Sweat\ rate}\, dt \qquad\qquad \text{(equation 3)}$$

The ratio is calculated because the onset and development of fever is normally proportional and directly linked to shivering, whereas fever can be assumed to be inversely proportional to sweat rate and drops when sweating. This is schematically depicted in FIG. 9, showing three fever index curves A-C progressing from a CBT having a normal or reference value 5 below an upper threshold 7 of such normal values to a strong fever value 6 above a critical threshold 8 as signaled by a shivering episode 52 and returning back to the normal or reference value 5 (as signaled by a sweating episode 54).

From the surrogate fever index curves A-C, time constants $\tau_{up}$ or $\tau_{down}$ can be determined or derived, in which, for example, $\tau_{up}$ is defined as the amount of time it takes for a surrogate fever curve 50 when starting from the reference value 5 to pass the critical threshold 8 and $\tau_{down}$ is defined as the amount of time it takes for a surrogate fever curve 50 when starting from the fever value 6 to pass the upper reference threshold 7. Alternatively, $\tau_{down}$ may be defined as the amount of time it takes for the fever to drop to 67% of its maximum value, similar to the calculation of decay times in electrical components such as capacitors. These time constants therefore carry clinical information about how fast fever develops and vanishes, e.g. after administration of medication. The surrogate fever curve as shown in FIG. 9 may be visualized on the user interface 41 or the user interface 42, for instance to enable clinical decision support for interventions or medication efficacy, e.g. to provide direct feedback to a medical caregiver on therapy response.

In an embodiment, the determination results of the shivering intensity and the sweat rate of the patient 1 may be scaled by the processor arrangement 34 based on available information regarding the thermal insulation of the patient 1. For example, the patient 1 during a shivering episode 52 may wear more clothes or add a blanket to keep warm, which can suppress the intensity of the shivering episode 52. Similarly, during a sweating episode 54, the patient 1 may shed clothing or blankets to cool down, thereby suppressing his or her sweat rate. To this end, the sensor arrangement 20 may provide information pertaining to a degree of thermal insulation of the patient to allow the processor arrangement 20 to determine at least one of a duration and a severity of each shivering episode 52 and sweating episode 54 as explained above as a function of the information pertaining to a degree of thermal insulation of the patient. Such information may be provided in any suitable manner. For example, determining the presence of a number blankets or clothing items, and/or the thickness of such blankets or clothing items could be derived from the image data provided by the camera 21. Alternatively, such information may be generated with the wearable sensor 22, for example by providing measurements of one or more of skin temperature, pressure, and ambient light, where in particular the pressure of a blanket pressing on the skin and ambient light, i.e. the light penetrating through the blanket and clothes, are independent of the core body temperature and are particularly useful parameters to monitor for this purpose.

The embodiments of the present invention can provide clinical insights into the thermoregulation capability of the body of the patient 1, e.g. to provide insights into the effect of administered medication, the severity of the disease, or assist in diagnosing the cause of fever. Such clinical insights can be useful in a number of care settings such as a hospital, hospital-to-home, or for home use, where it can assist in finding the right medication, dose, and timing, e.g. the clinical information provided by the processor arrangement 34 can aid the decision as to whether or not antibiotics are required to treat the medical condition of the patient 1, thereby reducing the risk that such antibiotics are administered unnecessarily, which is undesirable in the context of building up resistance to such antibiotics. However, it should be understood that use of the invention is not limited to medical care applications and may be utilized to get insight into the thermoregulation characteristics of the body of any patient, e.g. human or non-human, in general. In this context, the term patient as used in this application should not be interpreted as being limited to someone suffering from disease as the term is intended to apply to any patient of which the CBT is monitored in accordance with the teachings of the present application.

The above described embodiments of the method 100 executed by the processor arrangement 34 may be realized by computer readable program instructions embodied on a computer readable storage medium having, when executed on a processor arrangement 34 of a computing device 30, e.g. a patient monitoring terminal or the like, cause the processor arrangement 34 to implement any embodiment of the method 100. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In some embodiments, at least part of the computer readable program instructions may be incorporated in the processor arrangement 34 in the form of hardware.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. Measures recited in mutually different dependent claims can be advantageously combined.

**Claims**

1.  A patient monitoring system (10) for monitoring progression of a fever in a subject, comprising a processor arrangement (34) configured to:

    receive patient monitoring data from a sensor arrangement (20) for monitoring shivering and sweating of the patient as indicators of progression of a fever in the patient over the time interval;
    process the received patient monitoring data to detect shivering and sweating episodes (52, 54) and **characterized in that** the processor arrangement is further configured to:

    record the respective points in time at which the shivering episodes and sweating episodes occur; and construct (115, 117) a surrogate fever curve based on an alternating pattern of the shivering episodes and sweating episodes from the recorded respective points in time of the respective shivering and sweating episodes, the surrogate fever curve being for use in monitoring progression of fever in said subject.

2.  The patient monitoring system (10) of claim 1, wherein the processor arrangement is configured to:

    determine (153) a periodicity of the alternating pattern of the detected shivering and sweating episodes (52, 54) based on their respective occurrence at different points in time during the time interval; and identify a type of fever from the determined periodicity of the alternating pattern.

3.  The patient monitoring system (10) of claim 1 or 2, wherein the processor arrangement is configured to: determine at least one of a duration and an intensity of each shivering episode and sweating episode.

4.  The patient monitoring system (10) of claim 3, wherein the patient monitoring data comprises information pertaining to a degree of thermal insulation of the patient, and wherein the processor arrangement is configured to: determine at least one of a duration and a severity of each shivering episode and sweating episode as a function of the information pertaining to a degree of thermal insulation of the patient.

5.  The patient monitoring system (10) of any of claims 1-4, wherein the processor arrangement is configured to: determine a shivering frequency for each shivering episode (52).

6.  The patient monitoring system (10) of any of claims 1-5, wherein the processor arrangement is configured to: identify a sweating episode (54) based on a sweat rate of the patient.

7.  The patient monitoring system (10) of claim 6, wherein the processor arrangement is configured to:

determine (107) a sweat base rate for the patient from the received patient monitoring data during a shivering episode (52); and

detect a sweating episode (54) by detecting an actual sweat rate of the patient in the received patient monitoring data that exceeds the sweat base rate by a defined amount.

8. The patient monitoring system (10) of claim 6 or 7, wherein the processor arrangement is configured to:

compare an actual sweat rate of the patient against a defined sweat rate threshold; and

detect a sweating episode upon the actual sweat rate exceeding the defined sweat rate threshold.

9. The patient monitoring system (10) of any of claims 6-8, wherein the processor arrangement is configured to:

determine a signal strength of a signal pertaining to shivering episodes (52) over the time interval;

determine a total amount of sweat produced by the patient during the sweating episodes (54) over the time interval;

determine the patient's sweat rate over the time interval from the determined total amount of sweat produced by the patient;

calculate a ratio of the determined signal strength and determined sweat rate; and

output calculated ratio.

10. The patient monitoring system (10) of any of claims 1-9, wherein the patient monitoring data further comprises core body temperature data pertaining to a core body temperature of the patient measured over the time interval with a core body temperature sensor of the sensor arrangement, and wherein the processor arrangement is configured to output the core body temperature data.

11. The patient monitoring system (10) of any of claims 1-10, wherein the processor arrangement is configured to:

receive auxiliary information during the time interval through the communication interface arrangement, the auxiliary information comprising at least one of an ambient temperature to which the patient is exposed and a measurement of an activity level of the patient;

determine whether a shivering episode or a sweating episode is a reliable indicator of an illness-induced body temperature change in the patient based on the actual auxiliary information at the point in time of the shivering episode or sweating episode; and

output a shivering episode or a sweating episode only when the shivering episode or a sweating episode is determined to be a reliable indicator of progression of a fever in the patient.

12. The patient monitoring system (10) of claim 11, further comprising a communication interface arrangement (32) arranged to receive the input data in the form of patient monitoring data from a sensor arrangement (20) for monitoring shivering and sweating of the patient as indicators of progression of a fever in the patient (1) and to forward the patient monitoring data to the processor arrangement (34), the monitoring system optionally comprising the sensor arrangement.

13. A computer program product comprising a computer readable storage medium having computer readable program instructions embodied therewith for, when executed on a processor arrangement (34) of the patient monitoring system (10) of any one of claims 1 to 12, causing the processor arrangement to implement a patient monitoring method (100) comprising:

receiving the patient monitoring data from the sensor arrangement (20) for monitoring shivering and sweating of the patient as indicators of progression of a fever in the patient over the time interval;

processing the received patient monitoring data to detect shivering and sweating episodes (52, 54) and record the respective points in time at which the shivering episodes and sweating episodes occur; and

constructing (115, 117) a surrogate fever curve based on an alternating pattern of the shivering episodes and sweating episodes from the recorded respective points in time of the respective shivering and sweating episodes, the surrogate fever curve being for use in monitoring progression of fever in said subject.

**Patentansprüche**

1. Patientenüberwachungssystem (10) zum Überwachen eines Fieberverlaufs bei einem Subjekt, das eine Prozessoranordnung (34) umfasst, die dazu konfiguriert ist:

   Patientenüberwachungsdaten von einer Sensoranordnung (20) zum Überwachen von Schüttelfrost und Schweiß des Patienten als Indikatoren für einen Fieberverlauf beim Patienten über das Zeitintervall zu überwachen;
   die empfangenen Patientenüberwachungsdaten zu verarbeiten, um Schüttelfrost- und Schweißepisoden (52, 54) zu erkennen, und **dadurch gekennzeichnet, dass** die Prozessoranordnung weiter dazu konfiguriert ist:

   die jeweiligen Zeitpunkte aufzuzeichnen, zu welchen Schüttelfrost- und Schweißepisoden auftreten; und
   eine Ersatzfieberkurve auf der Grundlage eines abwechselnden Musters der Schüttelfrostepisoden und Schweißepisoden aus den jeweiligen aufgezeichneten Zeitpunkten der jeweiligen Schüttelfrost- und Schweißepisoden zu erstellen (115, 117), wobei die Ersatzfieberkurve zum Überwachen des Fieberverlaufs bei dem Subjekt verwendet wird.

2. Patientenüberwachungssystem (10) nach Anspruch 1, wobei die Prozessoranordnung dazu konfiguriert ist:

   eine Periodizität des abwechselnden Musters der erkannten Schüttelfrost- und Schweißepisoden (52, 54) auf der Grundlage ihres jeweiligen Auftretens zu unterschiedlichen Zeitpunkten während des Zeitintervalls zu bestimmen (153); und
   anhand der ermittelten Periodizität des abwechselnden Musters eine Fieberart zu identifizieren.

3. Patientenüberwachungssystem (10) nach Anspruch 1 oder 2, wobei die Prozessoranordnung dazu konfiguriert ist:
   mindestens eines von einer Dauer und einer Intensität jeder Schüttelfrostepisode und Schweißepisode zu bestimmen.

4. Patientenüberwachungssystem (10) nach Anspruch 3, wobei die Patientenüberwachungsdaten Informationen über einen Wärmeisolierungsgrad des Patienten umfassen und wobei die Prozessoranordnung dazu konfiguriert ist:
   mindestens eines von einer Dauer und/oder einem Schweregrad jeder Schüttelfrostepisode und Schweißepisode als Funktion der Informationen bezüglich eines Wärmeisolierungsgrads des Patienten zu bestimmen.

5. Patientenüberwachungssystem (10) nach einem der Ansprüche 1-4, wobei die Prozessoranordnung dazu konfiguriert ist:
   eine Schüttelfrostfrequenz für jede Schüttelfrostepisode (52) zu bestimmen.

6. Patientenüberwachungssystem (10) nach einem der Ansprüche 1-5, wobei die Prozessoranordnung dazu konfiguriert ist:
   eine Schweißepisode (54) basierend auf einer Schweißrate des Patienten zu bestimmen.

7. Patientenüberwachungssystem (10) nach Anspruch 6, wobei die Prozessoranordnung dazu konfiguriert ist:

   eine Schweißbasisrate für den Patienten aus den empfangenen Patientenüberwachungsdaten während einer Schüttelfrostepisode (52) zu bestimmen (107); und
   eine Schweißepisode (54) durch Erkennen einer tatsächlichen Schweißrate des Patienten in den empfangenen Patientenüberwachungsdaten, die die Schweißbasisrate um einen definierten Betrag überschreitet, zu erkennen.

8. Patientenüberwachungssystem (10) nach Anspruch 6 oder 7, wobei die Prozessoranordnung dazu konfiguriert ist:

   eine tatsächliche Schweißrate des Patienten mit einem definierten Schweißratenschwellenwert zu vergleichen; und
   eine Schweißepisode zu erkennen, wenn die tatsächliche Schweißrate den definierten Schweißratenschwellenwert überschreitet.

9. Patientenüberwachungssystem (10) nach einem der Ansprüche 6-8, wobei die Prozessoranordnung dazu konfiguriert ist:

eine Signalstärke eines Signals, das zu den Schüttelfrostepisoden (52) über das Zeitintervall gehört, zu bestimmen;

eine Gesamtmenge an Schweiß, die der Patient während der Schweißepisoden (54) über das Zeitintervall produziert, zu bestimmen;

die Schweißrate des Patienten über das Zeitintervall aus der ermittelten Gesamtmenge an Schweiß, die der Patient produziert, zu bestimmen;

ein Verhältnis der ermittelten Signalstärke und der ermittelten Schweißrate zu berechnen; und

ein berechnetes Ausgabeverhältnis auszugeben.

10. Patientenüberwachungssystem (10) nach einem der Ansprüche 1-9, wobei die Patientenüberwachungsdaten weiter Körperkerntemperaturdaten umfassen, die sich auf eine über das Zeitintervall mit einem Körperkerntemperatursensor der Sensoranordnung gemessene Körperkerntemperatur des Patienten beziehen, und wobei die Prozessoranordnung dazu konfiguriert ist, die Körperkerntemperaturdaten auszugeben.

11. Patientenüberwachungssystem (10) nach einem der Ansprüche 1-10, wobei die Prozessoranordnung dazu konfiguriert ist:

Zusatzinformationen während des Zeitintervalls über die Kommunikationsschnittstellenanordnung zu empfangen, wobei die Zusatzinformationen mindestens eine von einer Umgebungstemperatur, der der Patient ausgesetzt ist, und einer Messung eines Aktivitätsniveaus des Patienten umfassen;

basierend auf den tatsächlichen Zusatzinformationen zum Zeitpunkt einer Schüttelfrostepisode oder einer Schweißperiode zu bestimmen, ob eine Schüttelfrostepisode oder eine Schweißepisode ein zuverlässiger Indikator für eine krankheitsbedingte Veränderung der Körpertemperatur des Patienten ist; und

eine Schüttelfrostepisode oder eine Schweißepisode nur dann auszugeben, wenn die Schüttelfrostepisode oder eine Schweißepisode als zuverlässiger Indikator für einen Fieberverlauf bei dem Patienten gilt.

12. Patientenüberwachungssystem (10) nach Anspruch 11, das weiter eine Kommunikationsschnittstellenanordnung (32) umfasst, die dazu angeordnet ist, die Eingabedaten in Form von Patientenüberwachungsdaten von einer Sensoranordnung (20) zum Überwachen von Schüttelfrost und Schweiß des Patienten als Indikatoren für einen Fieberverlauf beim Patienten (1) zu empfangen und die Patientenüberwachungsdaten an die Prozessoranordnung (34) weiterzuleiten, wobei das Überwachungssystem optional die Sensoranordnung umfasst.

13. Computerprogrammprodukt, das ein computerlesbares Speichermedium umfasst, das darin computerlesbare Programmanweisungen aufweist, die, wenn sie auf der Prozessoranordnung (34) des Patientenüberwachungssystem (10) nach einem der Ansprüche 1 bis 12 ausgeführt werden, bewirken, dass die Prozessoranordnung ein Patientenüberwachungsverfahren (100) implementiert, das Folgendes umfasst:

Empfangen der Patientenüberwachungsdaten von der Sensoranordnung (20) zum Überwachen von Schüttelfrost und Schweiß des Patienten als Indikatoren für einen Fieberverlauf beim Patienten über das Zeitintervall;

Verarbeiten der empfangenen Patientenüberwachungsdaten, um Schüttelfrost- und Schweißepisoden (52, 54) zu erkennen und die jeweiligen Zeitpunkte aufzuzeichnen, zu denen die Schüttelfrostepisoden und Schweißepisoden auftreten; und

Erstellen (115, 117) einer Ersatzfieberkurve auf der Grundlage eines abwechselnden Musters der Schüttelfrostepisoden und Schweißepisoden aus den jeweiligen aufgezeichneten Zeitpunkten der jeweiligen Schüttelfrost- und Schweißepisoden, wobei die Ersatzfieberkurve zum Überwachen eines Fieberverlaufs bei dem Subjekt verwendet wird.

**Revendications**

1. Système de surveillance de patient (10) permettant de surveiller la progression d'une fièvre chez un sujet, comprenant un agencement de processeur (34) configuré pour :

recevoir des données de surveillance de patient en provenance d'un agencement de capteur (20) pour surveiller les frissons et la transpiration du patient en tant qu'indicateurs de la progression d'une fièvre chez le patient au cours de l'intervalle de temps ;

traiter les données de surveillance de patient reçues pour détecter des épisodes de frissons et de transpiration (52, 54) et **caractérisé en ce que** l'agencement de processeur est en outre configuré pour :

enregistrer les moments respectifs auxquels se produisent les épisodes de frissons et de transpiration ; et construire (115, 117) une courbe de fièvre de substitution basée sur un motif alterné des épisodes de frissons et des épisodes de transpiration à partir des moments respectifs enregistrés des épisodes de frissons et de transpiration respectifs, la courbe de fièvre de substitution étant destinée à être utilisée pour surveiller la progression de la fièvre chez ledit sujet.

2. Système de surveillance de patient (10) selon la revendication 1, dans lequel l'agencement de processeur est configuré pour :

déterminer (153) une périodicité du motif alterné des épisodes de frissons et de transpiration détectés (52, 54) sur la base de leur occurrence respective à différents moments pendant l'intervalle de temps ; et identifier un type de fièvre à partir de la périodicité déterminée du motif alterné.

3. Système de surveillance de patient (10) selon la revendication 1 ou 2, dans lequel l'agencement de processeur est configuré pour :
déterminer au moins l'une d'une durée et d'une intensité de chaque épisode de frissons et de transpiration.

4. Système de surveillance de patient (10) selon la revendication 3, dans lequel les données de surveillance de patient comprennent des informations se rapportant à un degré d'isolation thermique du patient et dans lequel l'agencement de processeur est configuré pour :
déterminer au moins l'une d'une durée et d'une gravité de chaque épisode de frissons et de transpiration en fonction des informations se rapportant au degré d'isolation thermique du patient.

5. Système de surveillance de patient (10) selon l'une quelconque des revendications 1-4, dans lequel l'agencement de processeur est configuré pour :
déterminer une fréquence de frissons pour chaque épisode de frissons (52).

6. Système de surveillance de patient (10) selon l'une quelconque des revendications 1-5, dans lequel l'agencement de processeur est configuré pour :
identifier un épisode de transpiration (54) sur la base d'un débit sudoral du patient.

7. Système de surveillance de patient (10) selon la revendication 6, dans lequel l'agencement de processeur est configuré pour :

déterminer (107) un débit sudoral de base pour le patient à partir des données de surveillance de patient reçues pendant un épisode de frissons (52) ; et détecter un épisode de transpiration (54) en détectant un débit sudoral réel du patient dans les données de surveillance de patient reçues qui dépasse le débit sudoral de base d'une quantité définie.

8. Système de surveillance de patient (10) selon la revendication 6 ou 7, dans lequel l'agencement de processeur est configuré pour :

comparer un débit sudoral réel du patient à un seuil de débit sudoral défini ; et détecter un épisode de transpiration lorsque le débit sudoral réel dépasse le seuil de débit sudoral défini.

9. Système de surveillance de patient (10) selon l'une quelconque des revendications 6-8, dans lequel l'agencement de processeur est configuré pour :

déterminer une intensité de signal d'un signal se rapportant à des épisodes de frissons (52) sur l'intervalle de temps ; déterminer une quantité totale de sueur produite par le patient pendant les épisodes de transpiration (54) sur l'intervalle de temps ; déterminer le débit sudoral du patient sur l'intervalle de temps à partir de la quantité totale déterminée de sueur produite par le patient ; calculer un rapport entre l'intensité de signal déterminée et le débit sudoral déterminé ; et délivrer un rapport calculé.

10. Système de surveillance de patient (10) selon l'une quelconque des revendications 1-9, dans lequel les données de surveillance de patient comprennent en outre des données de température corporelle centrale se rapportant à une température corporelle centrale du patient mesurée sur l'intervalle de temps avec un capteur de température corporelle centrale de l'agencement de capteur et dans lequel l'agencement de processeur est configuré pour délivrer les données de température corporelle centrale.

11. Système de surveillance de patient (10) selon l'une quelconque des revendications 1-10, dans lequel l'agencement de processeur est configuré pour :

recevoir des informations auxiliaires pendant l'intervalle de temps au moyen de l'agencement d'interface de communication, les informations auxiliaires comprenant au moins l'une d'une température ambiante à laquelle le patient est exposé et d'une mesure d'un niveau d'activité du patient ;

déterminer si un épisode de frissons ou un épisode de transpiration est un indicateur fiable d'un changement de température corporelle induit par la maladie chez le patient sur la base des informations auxiliaires réelles au moment de l'épisode de frissons ou de l'épisode de transpiration ; et

délivrer un épisode de frissons ou un épisode de transpiration uniquement lorsque l'épisode de frissons ou un épisode de transpiration est déterminé comme étant un indicateur fiable de la progression d'une fièvre chez le patient.

12. Système de surveillance de patient (10) selon la revendication 11, comprenant en outre un agencement d'interface de communication (32) agencé pour recevoir les données d'entrée sous la forme de données de surveillance de patient en provenance d'un agencement de capteur (20) pour surveiller les frissons et la transpiration du patient en tant qu'indicateurs de progression d'une fièvre chez le patient (1) et pour transmettre les données de surveillance de patient à l'agencement de processeur (34), le système de surveillance comprenant éventuellement l'agencement de capteur.

13. Produit programme informatique comprenant un support de stockage lisible par ordinateur dans lequel des instructions de programme lisibles par ordinateur sont intégrées pour, lorsqu'elles sont exécutées sur un agencement de processeur (34) du système de surveillance de patient (10) selon l'une quelconque des revendications 1 à 12, amener l'agencement de processeur à mettre en œuvre un procédé de surveillance de patient (100) comprenant :

la réception des données de surveillance de patient en provenance de l'agencement de capteur (20) pour surveiller les frissons et la transpiration du patient en tant qu'indicateurs de la progression d'une fièvre chez le patient au cours de l'intervalle de temps ;

le traitement des données de surveillance de patient reçues pour détecter des épisodes de frissons et de transpiration (52, 54) et enregistrer les moments respectifs auxquels les épisodes de frissons et de transpiration se produisent ; et

la construction (115, 117) d'une courbe de fièvre de substitution basée sur un motif alterné des épisodes de frissons et des épisodes de transpiration à partir des moments respectifs enregistrés des épisodes de frissons et de transpiration respectifs, la courbe de fièvre de substitution étant destinée à être utilisée pour surveiller la progression de la fièvre chez ledit sujet.

**FIG. 1**



EP 4 255 289 B1

**FIG. 2**

**FIG. 3**

18

100

## FIG. 4

**FIG. 5**

**FIG. 6**

FIG. 7

```
                    ┌─────────┐
                    │   151   │
                    └─────────┘
                         │
                         ▼
                    ┌─────────┐
                    │   153   │
                    └─────────┘
                         │
                         ▼
┌─────────┐  N, <24h      ◇       N, >24h  ┌─────────┐
│   171   │◄────────────│ 155 │────────────►│   174   │
└─────────┘              ◇               └─────────┘
                         │ Y
                         ▼
                    ◇
              │ 157 │────── N ──────┐
                    ◇               │
                    │ Y            │
                    ▼               ▼
              ┌─────────┐     ┌─────────┐
              │   172   │     │   173   │
              └─────────┘     └─────────┘
```

**FIG. 8**

**FIG. 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180242850 A

- WO 2017172837 A1